(19) Europäisches Patentamt
European Patent Office
Office européen des brevets

(11) **EP 4 347 002 B1**

(12) **EUROPEAN PATENT SPECIFICATION**

(45) Date of publication and mention
of the grant of the patent:
**25.03.2026 Bulletin 2026/13**

(21) Application number: **22726769.7**

(22) Date of filing: **09.05.2022**

(51) International Patent Classification (IPC):
*A61N 1/372* *(2006.01)*     *A61B 5/00* *(2006.01)*
*A61B 5/0215* *(2006.01)*     *A61N 1/362* *(2006.01)*
*A61N 1/375* *(2006.01)*

(52) Cooperative Patent Classification (CPC):
**A61N 1/37288; A61B 5/0006; A61B 5/002;**
**A61B 5/0028; A61B 5/0031; A61B 5/0215;**
**A61B 5/4848;** A61N 1/362; A61N 1/37217;
A61N 1/3756

(86) International application number:
**PCT/EP2022/062411**

(87) International publication number:
**WO 2022/248198 (01.12.2022 Gazette 2022/48)**

(54) **COMMUNICATION SYSTEM FOR INTRA-BODY COMMUNICATION**

KOMMUNIKATIONSSYSTEM FÜR KOMMUNIKATION INNERHALB EINES KÖRPERS

SYSTÈME DE COMMUNICATION POUR COMMUNICATION INTRACORPORELLE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **26.05.2021 US 202163193285 P**
**01.07.2021 EP 21183021**

(43) Date of publication of application:
**10.04.2024 Bulletin 2024/15**

(73) Proprietor: **BIOTRONIK SE & Co. KG**
**12359 Berlin (DE)**

(72) Inventors:
• **HUEGERICH, Burkhard**
**Portland, Oregon 97202 (US)**
• **KIBLER, Andrew B.**
**Lake Oswego, Oregon 97035 (US)**
• **QU, Min**
**Portland, Oregon 97223 (US)**
• **STOTTS, Larry**
**Fulshear, Texas 77441 (US)**

(74) Representative: **Biotronik Corporate Services SE**
**Corporate Intellectual Property**
**Sieversufer 9**
**12359 Berlin (DE)**

(56) References cited:
**US-A1- 2012 209 342     US-A1- 2018 236 249**
**US-A1- 2020 337 563     US-A1- 2021 085 989**
**US-A1- 2021 106 833**

**Description**

The invention provides a communication system for intra-body communication according to claim 1. Embodiments of the invention are defined in the dependent claims. For better understanding of the invention the present disclosure provides also further examples of similar communication systems.

Within a communication system of the kind described herein a communication in between implantable medical devices shall be facilitated, such as cardiac stimulation devices (for example a pacemaker device or a cardioverter defibrillator), sensor devices, recording devices or other medical devices to be implanted into a patient for providing a therapeutic or diagnostic function. A communication system of this kind may also be denoted as Body Area Network (BAN) system.

An implantable medical device of the kind concerned herein may for example be a pacemaker, an implantable cardioverter defibrillator, a sensor device such as a bio-sensor for measuring a blood pressure, or a recording device such as a loop recorder to be subcutaneously implanted in a patient.

An implantable pacemaker may for example be subcutaneously implanted in a patient and may comprise leads carrying electrodes and extending from a generator unit of the pacemaker device into the patient's heart for example to provide a pacing action in the right ventricle of the heart. Alternatively, an implantable pacemaker device may be designed as a leadless pacemaker not comprising leads, but being directly implanted into the patient's heart, for example in the right ventricle in order to provide for a pacing action.

A cardioverter defibrillator may serve for monitoring and treating potentially life-threatening arrhythmias in a patient's heart, wherein a cardioverter defibrillator of this kind may for example be implanted subcutaneously and may comprise leads extending into the patient's heart in order to record signals and to inject stimulation energy into the patient's heart for example to provide an electric shock (defibrillation).

Sensor devices, such as pressure sensors, flow sensors, temperature sensors or the like, may for example be implanted into a blood vessel, such as a vein, in order to provide for a monitoring of relevant parameters in the context of providing a therapy.

A loop recorder is for example subcutaneously implanted and serves to continuously record information for example about cardiac activity, such as an ECG. A loop recorder may continuously loop its memory and may store particular portions of signals, such that recorded signals may be communicated to an external device for analyzing the signals and for providing a diagnosis.

There is a desire that medical devices implanted in a patient may communicate with each other in order to allow the medical devices to interact. For example, signals sensed by a pacemaker device or an implanted sensor device may be transmitted to a loop recorder such that the loop recorder may record such signals. In addition, a pacemaker device may receive signals from a sensor device implanted remotely from the pacemaker device in order to take sensing signals of the sensor device into account for controlling a pacing action in the patient's heart.

For establishing a communication, approaches exist to create an intra-body network (IBN) linking implanted medical devices with each other such that signals may be exchanged in between the implanted medical devices.

For example, EP 2 327 609 B1 describes an acoustic communication link in between implanted medical devices for exchanging information in between the implanted medical devices. The acoustic communication link is established to permit wireless communication between the implanted medical devices, wherein transmission parameters may be adapted, such as a sensitivity and a carrier frequency, in order to improve an existing communication link.

Implantable medical devices may generally sense data which shall be communicated to another implantable medical device and in addition to an external device for further processing. Alternatively or in addition, implantable medical devices may receive configuration data from an external device in order to adapt operation of a particular implantable medical device. For this, a communication needs to be established with the external device.

In some approaches, a dedicated implantable medical device serves as a hub to provide a communication link to an external device with a dedicated communication concept differing from a signaling employed within the environment of the implantable medical devices. This comes with the drawback that the implantable medical device serving as the hub may exhibit an increased energy consumption, as communication with the external device may be power intensive. In addition, communication with the external device depends on the correct functioning of the implantable medical device serving as the hub, the hub having to implement different communication concepts in order to be able to establish a communication with the external device as well as with other implantable medical devices. Any failure of the dedicated implantable medical device serving as the hub may eliminate communication to the other implantable medical devices.

US 2006/0031378 A1 describes a system and method for providing digital data communications over a wireless intra-body network. A physical protocol layer is logically defined with an identifier uniquely assigned to a plurality of implantable devices in an intra-body network. Functions are specified within the physical protocol layer to permit data exchange over a wireless interface. A slave implantable device is activated in response to an activation signal transmitted through the wireless interface by a master implantable device. A wireless communication link is established between the slave implantable device and the master implantable device upon matching of the identifier assigned to the slave implantable device. Data is communicated intra-bodily over the communications link.

US 2007/02083890 A1 describes an apparatus and method for an implanted sound sensor wirelessly communicating with an implantable medical device, or with an external monitoring device.

In document US 2021/0106833 A1 a Technology for near infrared (NIR) based communication and/or sensing of implantable medical devices and systems as well as a method of operating the same are described.

In document US 2020/0337563 A1 a system for monitoring blood pressure is described including an implantable medical device (IMD) and an external device (ED). The IMD senses an electrogram (EGM) signal, identifies a feature thereof indicative of a ventricular depolarization, and transmits a conductive communication signal through patient tissue indicating when the ventricular depolarization occurred. The ED is worn against skin and configured to receive the conductive communication signal. The ED is also configured to sense a plethysmography (PG) signal and identify a feature thereof indicative of when a pulse wave responsive to the ventricular depolarization reaches a region of the patient adjacent the ED, and determine a delay time (TD) indicative of how long it takes the pulse wave to travel from the patient's heart to the region of the patient adjacent to the ED. The TD is a surrogate of the patient's blood pressure and useful for monitoring the patient's blood pressure and/or changes therein.

It is an object of the instant invention to provide a communication system which allows for a reliable, yet power efficient communication in between implantable medical devices as well as with an external device. It is a further object of the invention to provide intra-body communication with minimized energy consumption and maximized system simplicity to best support the requirements for small and low energy implantable devices.

In one aspect, a communication system for establishing an intra-body communication comprises: a multiplicity of implantable medical devices, each of the multiplicity of implantable medical devices comprising communication circuitry for communicating with another of the multiplicity of the implantable medical devices using a first signaling technique; and at least one external device comprising first communication circuitry for communicating with the multiplicity of implantable medical devices using said first signaling technique and second communication circuitry for communicating with a remote system using a second signaling technique different than the first signaling technique.

Accordingly, within the communication system multiple implantable medical devices are configured to communicate with each other and with an external device using a first signaling technique. Hence, each of the implantable medical devices is enabled to communicate with other implantable medical devices and an external device, i.e. a device outside of the patient's body.

Because the external device may communicate with any of the implantable medical devices (when implanted in a patient), no dedicated implantable medical device serving as a hub for relaying communication is required. Rather, a common communication technique is used for an intra-body communication in between the implantable medical devices as well as for a communication of the implantable medical devices with the external device.

By utilizing the same signaling technique for the intra-body communication between implantable medical devices (in an implanted state) amongst each other as well as for communication between the implantable medical devices and one or multiple external devices, the implementation complexity for communication circuitry of intra-body devices can be minimized. In addition, the external device is able to address individual implantable medical devices without the need of a specific intra-body hub to perform e.g. programming or interrogation tasks, and in addition to monitor a communication between the various medical devices to provide e.g. network maintenance tasks.

In addition, by allowing the communication to any individual internal implantable medical device by an external device, battery depletion or failure of an implantable medical device would not prevent the communication to other implantable medical devices.

The first communication circuitry of the at least one external device in particular is configured to communicate with each of the multiplicity of implantable medical devices using the first signaling technique. Hence, any implantable medical device is enabled to directly communicate with one or multiple external devices, without the need for a relaying by means of another implantable medical device.

The first signaling technique and the second signaling technique allow for a wireless communication, such that the communication system wirelessly spans medical devices implanted in a patient as well as external devices utilizing a common wireless signaling technique.

In one embodiment, the first signaling technique is based on a first physical layer signaling using modulated oscillating electrical fields. For example, the Intrabody Communication (IBC) technology may be used for a communication, as described in the IEEE 802.15.6 WBAN protocol. Herein, external devices may in particular couple to the patient using a galvanic coupling or a capacitive coupling.

In another embodiment, the first signaling technique is based on a physical layer signaling using acoustic signals, in particular ultrasonic signals. For this, communication circuitry of the implantable medical devices as well as the at least one external device may be formed as an ultrasonic transducer which is packaged such that it is vibrationally coupled to the medium surrounding the implantable medical device respectively the external device. An external device may for example be placed on the patient's skin in order to couple to the patient, such that ultrasonic signals may be transmitted into the patient and may be received from the patient.

In yet another embodiment, the first signaling technique is based on a physical layer signaling using oscillating magnetic fields or electromagnetic wave signals. In this embodiment, the implantable medical devices as well as the at least one external device comprise communication circuitry using magnetic coils and using modulated oscillating magnetic fields for communication in between the implantable medical devices as well as with the external device.

In another embodiment, the first signaling technique is based on a physical layer using RF technology with a carrier frequency suitable for penetrating body tissue.

Within the communication, a common physical layer signaling as well as a common communication protocol is used for communication in between the implantable medical devices and the at least one external device. The physical layer herein denotes a logical layer defining a physical signaling in between the different devices using a common technology according to a common communication protocol. Each implantable medical device as well as the at least one external device herein may be identified within communication messages by means of a unique identifier, such that messages transmitted through the patient's body are received by all devices and processed by the devices according to identifier information included in the messages.

In one embodiment, the at least one external device is configured to externally contact a patient. For example, the at least one external device may be configured to be worn on a patient's body. For example, the at least one external device may have the shape of a wrist watch and hence may be worn on a patient's wrist, wherein a contact face of the at least one external device is configured to be brought into contact with the patient's skin for coupling to the patient, for example for introducing electrical signals or acoustical signals (in particular ultrasonic signals) into the patient or receiving such signals from the patient.

The at least one external device in addition implements a second signaling technique, which for example is based on a second physical layer signaling using RF signals. The second signaling technique may for example employ a standardized communication technology, such as a Bluetooth technique, in particular Bluetooth Low Energy (BLE), a Zigbee technique, a Near Field Communication (NFC) technique, or a Wi-Fi technique.

The second signal technique implements a second (logical) physical layer for establishing a communication of the at least one external device with a remote system, such as a remote server accessible via a public communications network, such as the Internet. By means of the second signaling technique the at least one external device may e.g. connect to a communication device serving as a relay device, such as a local computer, a mobile device such as a smart phone or another local device in proximity to the patient, wherein by means of the second signaling technique in particular a communication within a local environment may be established. By means of the communication device data may be relayed to and from the remote system, for example using a common communication protocol scheme by means of the public communications network.

In one embodiment, the second signaling technique is based on a second physical layer using acoustic signals. For example, the second communication circuitry of the at least one external device may comprise an acoustic transducer which is configured to produce acoustic signals in an audible acoustic frequency range, for example in between 50 Hz to 8 kHz, which may be transmitted using a Public Switched Telephone Network (PSTN). For example, for relaying data to a remote system, a user may call a phone number using a telephone, the phone number being associated with a central server link station. The user may then place the at least one external device in proximity to the telephone's microphone such that a data transmission may take place by transferring the acoustic signals produced by the at least one external device via the telephone to the remote system, without requiring an Internet connection or other specialized hardware.

In one embodiment, at least one of the implantable medical devices is a cardiac stimulation device, such as a pacemaker device or a cardioverter defibrillator.

In one embodiment, at least one of the implantable medical devices is a leadless cardiac stimulation device, that is a cardiac stimulation device not comprising leads carrying electrodes. A leadless cardiac stimulation device of this kind may in particular be implanted directly into the patient's heart, for example the right ventricle or the right atrium.

Generally, one or multiple implantable medical devices of the communication system may be implanted subcutaneously into a patient or directly into the patient's heart. For example, in one embodiment, one implantable medical device is implanted into the right ventricle and another implantable medical device is implanted into the right atrium of the patient's heart, a communication being established in between the intra-cardiac implantable medical devices within the communication system.

In one embodiment, at least one of the implantable medical devices is a subcutaneous cardiac loop recorder or a bio-sensor for measuring the blood pressure.

Generally, within the communication system different implantable medical devices e.g. in the shape of stimulation devices, sensing devices, recording devices or marking devices may communicate with one another and with at least one external device making use of a common (first) signaling technique, involving a common physical layer as well as a common communication protocol.

In one embodiment, within the communication system an intra-cardiac pacemaker is implanted in the right ventricle and an intra-cardiac pacemaker is implanted in the right atrium.

In one embodiment, within the communication system an intra-cardiac pacemaker is implanted in the right ventricle and a loop recorder is implanted subcutaneously.

In one embodiment, within the communication system an intra-cardiac pacemaker is implanted in the right ventricle and a sensor (e.g. a bio sensor) measuring the blood pressure is implanted in a blood vessel of the patient and/or integrated into the intra-cardiac pacemaker.

The communication system may be based on energy saving optimizations using modulated oscillating electric fields to communicate between implanted devices within a body. Such communication concepts utilize high frequency electric pulses to either being phase-shift keying (PSK) or on-off keying (OOK) modulated to convey bit information. In either case the carrier frequency may be generated by a 50% duty cycle square pulse shaped signal. In case of a PSK modulation, the carrier frequency signal is continuous, and phase shifts of the carrier frequency are indicative for the change in the bit value transmitted. In case of an OOK modulation, the presence of the carrier frequency for a predetermined time represents the code for the bit value 1 and the absence of the carrier frequency, again for a predetermined time frame, represents the code for bit value 0. This basic principle of the OOK modulation makes it a good candidate for a simple communication concept with good energy saving potential.

There may be two energy saving parameter available for an OOK modulated oscillating electric field with a 50% duty cycle square pulse signal shape of the carrier frequency:

    1. the predetermined time used to define a bit duration (0 or 1), and
    2. the duty cycle used for the square pulse signal of the carrier frequency.

Tests indicate that the demodulation of OOK modulated oscillating electric fields may be performed for significantly reduced pulse width times together with a strong reduced duty cycle. Reducing the duty cycle for the active part of the pulse interval effectively produces a wideband signal. The narrower the pulse the wider the frequency spectrum will be. Applying both energy saving methods for the generation of the communication signal can yield a magnitude of energy savings in comparison to the existing state of the art technology. This can be observed by comparing the active signal time (AST) as used in existing technologies (e.g. ZAND communication) with test results utilizing energy optimized signal parameters. Since AST represents the time an active voltage is applied to the transmitting electrodes, reducing AST will directly reduce the energy needed to generate the transmission signal.

AST in OOK may apply by design only to bit transmissions of the value 1 where the predetermined bit time can be expressed in the numbers of carrier frequency intervals needed for that time. Finally, with the duty cycle of the square pulse shape further limiting the active signal time, AST can be expressed as

$$AST = (1/fc) * N * DC$$

with

    fc = carrier frequency,
    N = number of carrier frequency intervals, and
    DC = duty cycle of the carrier frequency square pulse shape.

For the communication with OOK modulation the number of intervals may be about 60 and the duty cycle used may be 0.5 (50%). For example, successful communication may be achieved with parameter N set to 10 and the duty cycle value set to 0.1 (10%), yield an energy reduction factor of 30 in comparison to the settings above.

The described embodiments above and any combination of them allow for implanted devices to perform communication with minimal energy consumption by utilizing none complex technology.

In another aspect, a method for operating a communication system for intra-body communication comprises: establishing a communication in between a multiplicity of implantable medical devices, each comprising communication circuitry for communicating with another of the multiplicity of implantable medical devices using a first signaling technique; establishing a communication in between at least one external device and the multiplicity of implantable medical devices, the at least one external device comprising first communication circuitry for communicating with the multiplicity of implantable medical devices using said first signaling technique; and establishing a communication between the at least one external device and a remote system, the at least one external device comprising second communication circuitry for communicating with the remote system using a second signaling technique different than the first signaling technique.

The advantages and advantageous embodiments described above for the communication system equally apply also to the method, such that it shall be referred to the above in this respect.

The various features and advantages of the present invention may be more readily understood with reference to the following detailed description and the embodiments shown in the drawings. Herein,

Fig. 1    shows a schematic drawing of a communication system of medical devices implanted in a patient;

Fig. 2    shows a schematic drawing of two medical devices in between which a communication link for a data communication in between the medical devices shall be established;

Fig. 3    shows a schematic drawing of a communication system including multiple medical devices implanted in a patient and a variety of external devices;

Fig. 4    shows a schematic drawing of a physical layer signaling in between the implantable medical devices, an external device and a remote system; and

Fig. 5    shows an example of a data transmission in between an external device and a remote system using an acoustic signaling technique.

Subsequently, embodiments of the invention shall be described in detail with reference to the drawings. In the drawings, like reference numerals designate like structural elements.

It is to be noted that the embodiments are not limiting for the invention, but merely represent illustrative examples.

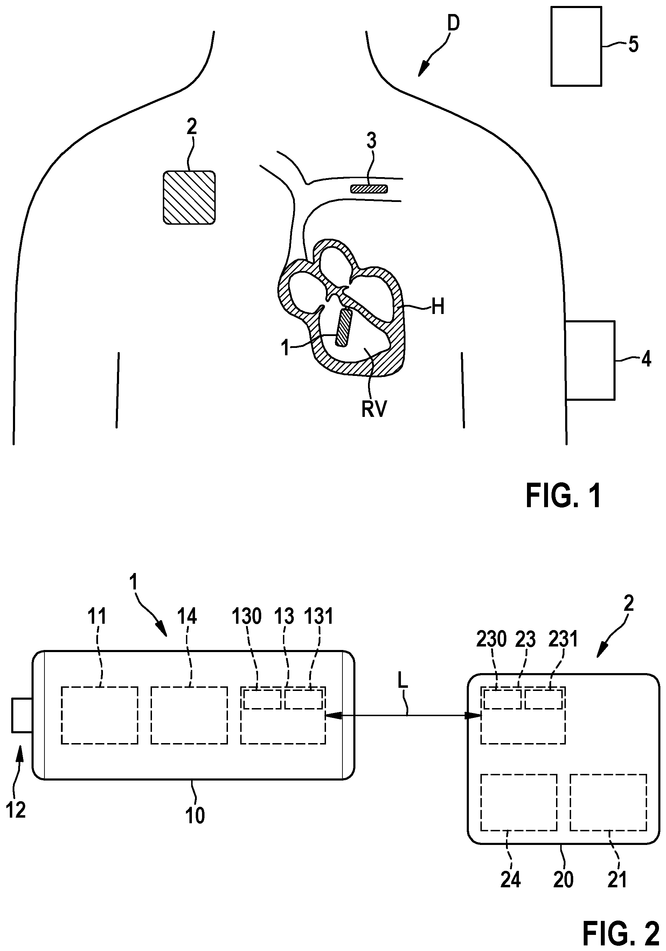

Referring to Fig. 1, implantable medical devices 1, 2, 3 may be implanted in a patient P at different locations in order to provide different functions within the patient P. For example, a medical device 1 in the shape of a leadless pacemaker device may be implanted in the right ventricle RV of the patient's heart H in order to provide for a pacing action within the heart H. Another medical device 2 for example in the shape of a loop recorder may subcutaneously be implanted within the chest, the loop recorder allowing for a recording of signals and, for example, a communication with an external device in order to monitor certain parameters within the patient P. Another implantable medical device 3 in the shape of a sensor device, for example a pressure sensor, a flow sensor or a temperature sensor or the like, may be implanted for example in a blood vessel in order to sense characteristic parameters such as a blood pressure or a blood flow.

There generally exists a desire for a data communication in between different medical devices 1, 2, 3 implanted in a patient P. Approaches exist to establish a communication of this kind in a wireless fashion by establishing an intra-body network linking the medical devices 1, 2, 3 together, such that data may be exchanged in between the medical device 1, 2, 3. A loop recorder may hence for example record sensor data of a sensor device, or data of a pacemaker or a cardioverter defibrillator, and may also provide data for example to a pacemaker or a cardioverter defibrillator to control a therapeutic action.

In order to allow for a data communication, a communication link between medical devices 1, 2, 3 needs to be established. Signals herein are exchanged in a modulated fashion making use of a particular transmission technology, such as an acoustic, electric or magnetic (inductive) signal transmission, and a particular modulation scheme, such as a PCM, FSK, PSK, QPSK, FM, or AM modulation or the like.

Referring now to Fig. 2, medical devices 1, 2 to be implanted in a patient P may have a small built and may be designed for a low power consumption in order to remain in a patient P over a long-term.

A first medical device 1, for example in the shape of a leadless pacemaker, may herein comprise a housing 10, a control circuitry 11, an electrode arrangement 12 for emitting stimulation signals or receiving sense signals, a communication circuitry 13 and an energy storage 14, for example in the shape of a battery.

A second medical device 2, for example in the shape of an implantable sensor device, such as a pressure sensor, or in the shape of a loop recorder, comprises a housing 20, a control circuitry 21, a communication circuitry 23, and an energy storage 24, for example in the shape of a battery.

The communication circuitry 13, 23, in each case, comprises a transmission unit 130, 230, and a reception unit 131, 231. The communication circuitry 13, 23, is designed for the specific transmission technology, that is for transmitting and receiving acoustic, electric or magnetic signals. Also, the communication circuitry 13, 23 is designed to modulate respectively demodulate signals for transmission and reception, to optimize transmission parameters, to amplify received signals and to process signals in order to forward processed signals to the control circuitry 11, 21 for an analysis and control of the operation of the medical device 1, 2.

Generally, within a communication system as schematically shown in Fig. 1, a communication in between implantable medical devices 1, 2, 3 shall be established using communication links. Referring for example to Fig. 2, a communication link L shall be established in between the medical devices 1, 2 for example at the initial startup of one of the medical devices 1, 2 or after exiting a sleep mode after a prolonged duration of passivity of the medical device 1, 2. Herein, a medical device 1, 2 which wishes to establish a communication may for example send out a trigger signal towards the other medical device 2, 1, the trigger signal indicating that the medical device 1 wishes to establish communication. Communication then takes place using a common physical layer signaling employing a signaling technology utilizing for example electrical, acoustic or magnetic signals.

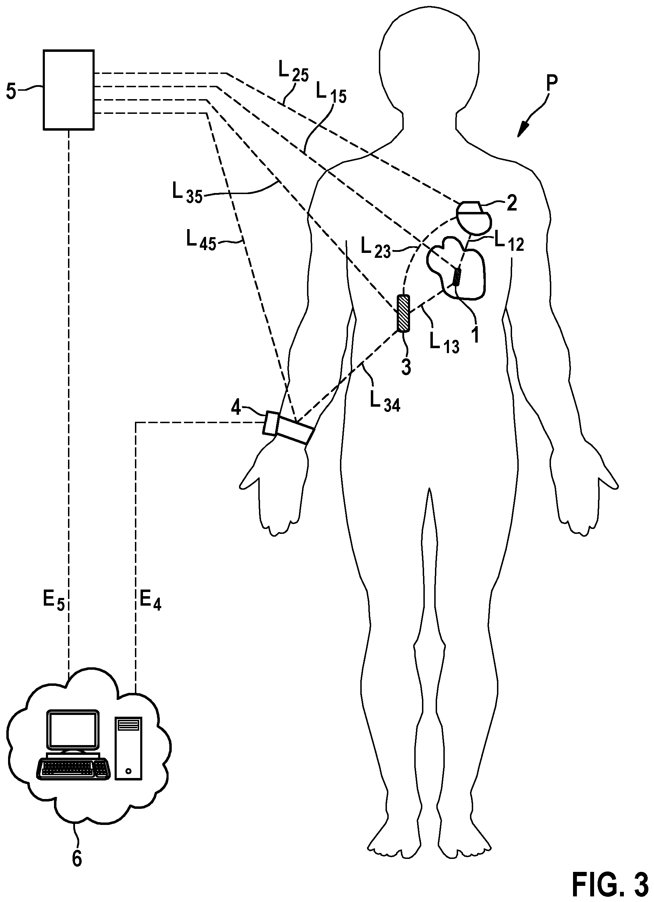

Referring now to Fig. 3, medical devices 1, 2, 3 implanted in a patient P may establish a communication for transmitting data amongst each other using communication links $L_{12}$, $L_{13}$, $L_{23}$. Via such communication links

$L_{12}$, $L_{13}$, $L_{23}$ for example measurement data may be transferred from a sensing device to a recording device, such as a loop recorder, or to a stimulation device, for processing by the receiving device.

In addition, the medical devices 1, 2, 3 implanted in the patient P may communicate with one or multiple external devices 4, 5 in order to transfer data, for example measurement data, from the medical device 1, 2, 3 towards the external device 4, 5, or to receive configuration data from the external device 4, 5 to adapt operation of the implanted medical device 1, 2, 3. Communication herein takes place via communication links $L_{15}$, $L_{25}$, $L_{35}$, $L_{34}$ connecting the external devices 4, 5 and the implanted medical devices 1, 2, 3 with each other, wherein in addition the external devices 4, 5 may also communicate with each other via a communication link $L_{45}$.

In order to facilitate communication in between the intra-body medical devices 1, 2, 3 and the external devices 4, 5, it herein is proposed to use a common communication technology for both the internal, intrabody devices 1, 2, 3 and the external devices 4, 5, such that the medical devices 1, 2, 3 may communicate with each other as well as with the external devices 4, 5 using the same communication technology.

The common communication technology in particular involves a common physical layer signaling and a common communication protocol.

For example, the implantable medical devices 1, 2, 3 and the external devices 4, 5 may use a signaling technique based on a physical layer signaling using modulated oscillating electric fields. For example, a signaling according to the standardized Intra-Body Communication (IBC) may be employed, using a galvanic coupling or a capacitive coupling of the external devices 4, 5 to the patient P.

In another embodiment, the implantable medical devices 1, 2, 3 and the external devices 4, 5 may use a signaling technique based on a physical layer signaling using acoustic signals, in particular ultrasonic signals. In this case, the implantable medical devices 1, 2, 3 and the external devices 4, 5 employ an acoustic transducer in order to couple to surrounding medium. In particular, the implantable medical devices 1, 2, 3 may employ a transducer contacting tissue. The external devices 4, 5 may be placed on the patient's skin, as it for example is the case for the external device 4 having the shape of a wrist watch in the embodiment of Fig. 3.

In yet another embodiment, the implantable medical devices 1, 2, 3 and the external devices 4, 5 may use a signaling technique based on a physical layer signaling using modulated oscillating magnetic fields. In this case the medical devices 1, 2, 3 and the external devices 4, 5 comprise a transducer in the shape of a magnetic coil to induce oscillating magnetic fields.

In yet another embodiment, the implantable medical devices 1, 2, 3 and the external devices 4, 5 may use a signaling technique based on a physical layer signaling using modulated electromagnetic waves. In this case the medical devices 1, 2, 3 and the external devices 4, 5 comprise a transducer in the shape of a suitable antenna.

One or multiple of the external devices 4, 5 may be placed on the patient's skin and for this may for example be worn by the patient P, as it is the case for the external device 4 in the shape of the wrist watch in the embodiment of Fig. 3.

Communication in between the implantable medical devices 1, 2, 3 amongst each other, in between the implantable medical devices 1, 2, 3 and the external device 4, 5, and in between the external devices 4, 5 amongst each other using the common signaling technique is based on a transmission of modulated signals, wherein the signals may encode identifier information identifying and addressing devices 1-5. Each device 1-5 herein may be identified by a unique identifier, such that messages can be exchanged in between the devices 1-5.

The communication links as illustrated in Fig. 3 hence are established as logical links, signals generally being transmitted using the body of the patient P as a wave guiding medium, such that signals transmitted from one device 1-5 generally are received by all devices 1-5 coupled within the network, but may be discerned according to identifier information encoded in the signals.

In the embodiment of Fig. 3, the external devices 4, 5 are configured to communicate with a remote system 6, for example a remote server system within a public communications network, such as the Internet. For example, data may be transmitted from one or both of the external devices 4, 5 to the remote system 6, and may be received from the remote system 6 at the external devices 4, 5. The remote system 6 communicates with the external device 4 via the external link E4 and with the external device 5 via the external link E5.

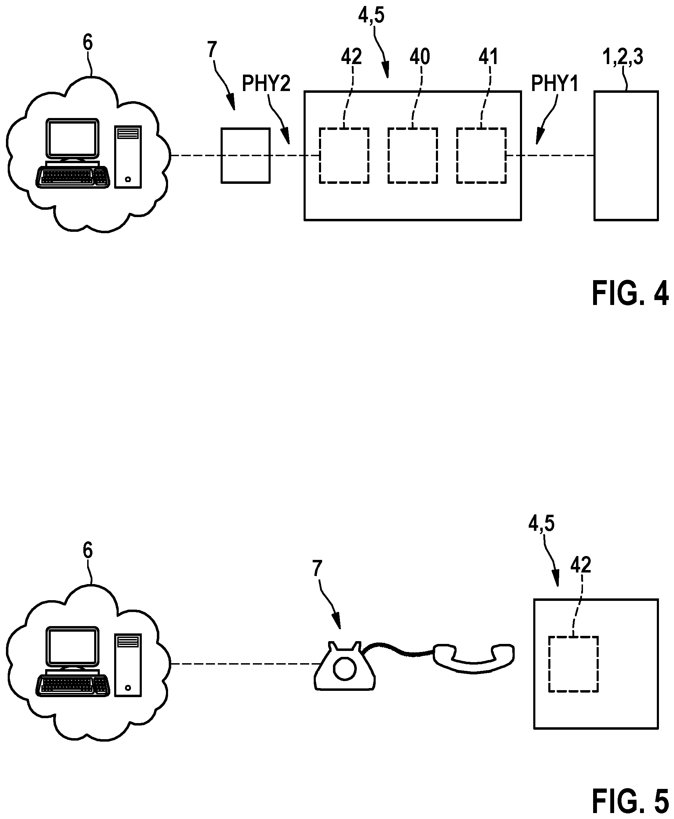

Referring now to Fig. 4, each of the external devices 4, 5 comprises first communication circuitry 41 for establishing a communication with the implantable medical devices 1, 2, 3 making use of a common physical layer signaling PHY1. By means of the common physical layer signaling PHY1 a communication may be established in between the external devices 4, 5 to any of the implantable medical devices 1, 2, 3 individually, such that data may be exchanged between the external devices 4, 5 and any of the implantable medical devices 1, 2, 3.

In addition, the external device 4, 5 comprises a second communication circuitry 42 for establishing a communication with the remote system 6 using a physical layer signaling PHY2 different from the physical layer signaling PHY1. By means of the communication circuitry 42 data may be exchanged with the remote system 6.

The physical layer signaling PHY2 may for example utilize a standard close-proximity communication scheme such as Bluetooth, Zigbee, Near Field Communication (NFC), or Wi-Fi. In order to establish a communication to the remote system 6, herein a connection using the physical layer signaling PHY2 may be established to a local communication device 7, such as a mobile device or a local computer, which relays the data and communicates with a remote server 6 via a public communication network. The remote systems 6 can be the remote server 6 and vice versa.

A control unit 40 serves to control operation of the external device 4, 5 and processes data. For example, the control unit 40 may be configured to translate data between the different communication circuitry 41, 42, for transmission in between the remote server 6 and the implantable medical devices 1, 2, 3.

Referring now to Fig. 5, in one embodiment the local communication device 7 may have the shape of a regular telephone, which is connected to the remote system 6 via a Public Switched Telephone Network (PSTN). The communication circuitry 42 of the external device 4, 5 may for example be configured to produce acoustic signals in an audible frequency range, for example in between 50 Hz and 8 kHz, which may be transferred via a regular telephone line. To establish a communication to the remote system 6, a user may for example dial a phone number associated with a central server link station of the remote system 6, upon which the user may place the external device 4, 5 in proximity to the telephone's microphone such that data can be transmitted using acoustic signals via the communication device 7 in the shape of the telephone towards the remote system 6.

The implantable medical devices 1, 2, 3 within the communication system may for example be cardiac stimulation devices, such as a leadless pacemaker devices or cardioverter defibrillators, sensing devices such as biosensors for example for sensing a blood pressure, or recording devices such as a loop recorder.

Generally, the implantable medical devices 1, 2, 3 may be implanted fully or in part directly into the patient's heart, for example the right ventricle or the right atrium. In another embodiment, the implantable medical devices 1, 2, 3 may be implanted fully or in part subcutaneously within the patient.

As a communication between the external devices 4, 5 and the implantable medical devices 1, 2, 3, in the above embodiment, may be established possibly only by bringing the external devices 4, 5 into close proximity with the patient P, beneficially in contact with the patient's skin, inherently an increased security is achieved, in that communication with the implantable medical devices 1, 2, 3 cannot easily be corrupted or interrupted.

## List of reference numerals

| 1, 2, 3 | Implantable medical device |
| 10, 20 | Housing |
| 11, 21 | Control circuitry |
| 12 | Electrode arrangement |
| 13, 23 | Communication circuitry |
| 130, 230 | Transmission unit |
| 131, 231 | Reception unit |
| 14, 24 | Energy storage |
| 4, 5 | External device |
| 40 | Control unit |
| 41 | Communication circuitry |
| 42 | Communication circuitry |
| 6 | Remote system (remote server) |
| 7 | Communication device |
| E4, E5 | External link |
| H | Heart |
| L | Communication link |
| $L_{12}$-$L_{45}$ | Communication links |
| P | Patient |
| PHY1, PHY2 | Physical layer signaling |
| RV | Right ventricle |

## Claims

1. A communication system for establishing an intra-body communication, comprising:

   a multiplicity of implantable medical devices (1-3), each of the multiplicity of implantable medical devices (1-3) comprising communication circuitry (13, 23) for communicating with another of the multiplicity of implantable medical devices (1-3) using a first signaling technique; and
   at least one external device (4, 5) comprising first communication circuitry (41) for communicating with the multiplicity of implantable medical devices (1-3) using said first signaling technique and second communication circuitry (42) for communicating with a remote system (6) using a second signaling technique different than the first signaling technique, wherein
   the first signaling technique is based on a first physical layer signaling using ultrasonic signals, wherein
   at least one of the multiplicity of implantable medical devices (1-3) is a leadless cardiac stimulation device.

2. The communication system according to claim 1, wherein the first communication circuitry (41) of the at least one external device (4, 5) is configured to communicate with each of the multiplicity of implantable medical devices (1-3) using said first signaling technique.

3. The communication system according to one of the preceding claims, wherein the at least one external device (4, 5) is configured to externally contact a patient.

4. The communication system according to one of the preceding claims, wherein the at least one external device (4, 5) is configured to be worn on a patient's body.

5. The communication system according to one of claims 1 to 4, wherein the second signaling technique is based on a second physical layer signaling using RF signals.

6. The communication system according to claim 5, wherein the second signaling technique is a standardized communication technology.

7. The communication system according to one of the preceding claims, comprising a communication device (7) to be coupled to the at least one external device (4, 5) for establishing a data transmission in between the at least one external device (4, 5) and the remote system (6) using the second signaling technique.

8. The communication system according to one of the preceding claims, wherein at least one of the multiplicity of implantable medical devices (1-3) is a subcutaneous cardiac loop recorder or a bio-sensor for measuring the blood pressure.

**Patentansprüche**

1. Kommunikationssystem zum Herstellen einer innerkörperlichen Kommunikation, umfassend:

eine Vielzahl implantierbarer medizinischer Geräte (1-3), wobei jedes der Vielzahl implantierbarer medizinischer Geräte (1-3) eine Kommunikationsschaltung (13, 23) zum Kommunizieren mit einem anderen der Vielzahl implantierbarer medizinischer Geräte (1-3) unter Verwendung einer ersten Signalübertragungstechnik umfasst; und
wenigstens ein externes Gerät (4, 5), das eine erste Kommunikationsschaltung (41) zum Kommunizieren mit der Vielzahl implantierbarer medizinischer Geräte (1-3) unter Verwendung der ersten Signalübertragungstechnik und eine zweite Kommunikationsschaltung (42) zum Kommunizieren mit einem Fernsystem (6) unter Verwendung einer zweiten Signalübertragungstechnik, die sich von der ersten Signalübertragungstechnik unterscheidet, umfasst, wobei
die erste Signalübertragungstechnik auf einer ersten physikalischen Schicht basiert, die unter Verwendung von Ultraschallsignalen Signale überträgt, wobei
es sich bei wenigstens einem der Vielzahl implantierbarer medizinischer Geräte (1-3) um ein leitungsloses Herzstimulationsgerät handelt.

2. Kommunikationssystem nach Anspruch 1, wobei die erste Kommunikationsschaltung (41) des wenigstens einen externen Geräts (4, 5) dazu ausgestaltet ist, mit jedem der Vielzahl implantierbarer medizinischer Geräte (1-3) unter Verwendung der ersten Signalübertragungstechnik zu kommunizieren.

3. Kommunikationssystem nach einem der vorstehenden Ansprüche, wobei das wenigstens eine externe Gerät (4, 5) dazu ausgestaltet ist, einen Patienten extern zu kontaktieren.

4. Kommunikationssystem nach einem der vorstehenden Ansprüche, wobei das wenigstens eine externe Gerät (4, 5) dazu ausgestaltet ist, am Körper eines Patienten getragen zu werden.

5. Kommunikationssystem nach einem der Ansprüche 1 bis 4, wobei die zweite Signalübertragungstechnik auf einer zweiten physikalischen Schicht basiert, die unter Verwendung von HF-Signalen Signale überträgt.

6. Kommunikationssystem nach Anspruch 5, wobei es sich bei der zweiten Signalübertragungstechnik um eine standardisierte Kommunikationstechnologie handelt.

7. Kommunikationssystem nach einem der vorstehenden Ansprüche, das ein Kommunikationsgerät (7) umfasst, das mit dem wenigstens einen externen Gerät (4, 5) gekoppelt werden soll, um eine Datenübertragung zwischen dem wenigstens einen externen Gerät (4, 5) und dem Fernsystem (6) unter Verwendung der zweiten Signalübertragungstechnik herzustellen.

8. Kommunikationssystem nach einem der vorstehenden Ansprüche, wobei es sich bei wenigstens einem der Vielzahl implantierbarer medizinischer Geräte (1-3) um einen subkutanen Herzschleifenrekorder oder einen Biosensor zum Messen des Blutdrucks handelt.

**Revendications**

1. Système de communication destiné à établir une communication intracorporelle, comprenant :

une multiplicité de dispositifs médicaux implantables (1 à 3), chacun de la multiplicité de dispositifs médicaux implantables (1 à 3) comprenant un circuit de communication (13, 23) destiné à communiquer avec un autre de la multiplicité de dispositifs médicaux implantables (1-3) en utilisant une première technique de signalisation ; et

au moins un dispositif externe (4, 5) comprenant un premier circuit de communication (41) destiné à communiquer avec la multiplicité de dispositifs médicaux implantables (1 à 3) en utilisant ladite première technique de signalisation et un second circuit de communication (42) destiné à communiquer avec un système distant (6) en utilisant une seconde technique de signalisation différente de la première technique de signalisation, dans lequel

la première technique de signalisation est basée sur une première signalisation de couche physique utilisant des signaux ultrasonores, dans lequel

au moins un de la multiplicité de dispositifs médicaux implantables (1 à 3) est un dispositif de stimulation cardiaque sans câbles.

2. Système de communication selon la revendication 1, dans lequel le premier circuit de communication (41) de l'au moins un dispositif externe (4, 5) est configuré pour communiquer avec chacun de la multiplicité de dispositifs médicaux implantables (1-3) en utilisant ladite première technique de signalisation.

3. Système de communication selon l'une des revendications précédentes, dans lequel l'au moins un dispositif externe (4, 5) est configuré pour entrer en contact externe avec un patient.

4. Système de communication selon l'une des revendications précédentes, dans lequel l'au moins un dispositif externe (4, 5) est configuré pour être porté sur le corps d'un patient.

5. Système de communication selon l'une des revendications 1 à 4, dans lequel la seconde technique de signalisation est basée sur une seconde signalisation de couche physique utilisant des signaux RF.

6. Système de communication selon la revendication 5, dans lequel la seconde technique de signalisation est une technologie de communication normalisée.

7. Système de communication selon l'une des revendications précédentes, comprenant un dispositif de communication (7) à coupler à l'au moins un dispositif externe (4, 5) pour établir une transmission de données entre l'au moins un dispositif externe (4, 5) et le système distant (6) en utilisant la seconde technique de signalisation.

8. Système de communication selon l'une des revendications précédentes, dans lequel au moins un de la multiplicité de dispositifs médicaux implantables (1 à 3) est un enregistreur sous-cutané de boucle cardiaque ou un biocapteur destiné à mesurer la pression artérielle.

**FIG. 1**

**FIG. 2**

FIG. 3

FIG. 4

FIG. 5

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2327609 B1
- US 20060031378 A1
- US 200702083890 A1
- US 20210106833 A1
- US 20200337563 A1